# EUROPÄISCHE PATENTSCHRIFT

(11) **EP 1 566 179 B1**
(45) Veröffentlichungstag und Bekanntmachung des Hinweises auf die Patenterteilung: **15.10.2008**
(21) Anmeldenummer: 05009586.8
(22) Anmeldetag: 01.07.2002
(51) Int. Cl.: A61K 36/8962, A61K 8/06, A61P 17/02

(54) **Fett(öl)haltiges Mittel, enthaltend Zwiebelextrakt, seine Herstellung und seine Verwendung**
Agent containing fat (oil) which contains onion extract, the production and use thereof
Agent contenant de la graisse (de l'huile) et de l'extrait d'oignon, sa production et son utilisation

(30) Priorität: 03.07.2001 DE 10132003
(43) Veröffentlichungstag der Anmeldung: 24.08.2005
(62) Teilanmeldung aus: 02760209.3
(73) Patentinhaber: Merz Pharma GmbH & Co. KGaA, 60318 Frankfurt Main (DE)
(72) Erfinder: Beutler, Rolf D., 64739 Höchst/Hummetroth (DE); Schatschneider, Simone, 65207 Wiesbaden (DE); Heberer, Martina, 63073 Offenbach (DE); Paspaleeva-Kühn, Valentina, 60323 Frankfurt/Main (DE)
(74) Vertreter: Müller, Claudia

(56) Entgegenhaltungen:
- EP-A- 0 273 407
- EP-A- 0 818 203
- WO-A-98/50054
- WO-A-02/055049
- FR-A- 2 681 531
- US-A- 5 885 581
- ROSSI A. ET AL: "[Treatment of keloid and hypertrophic scar with Kelimed.RTM. ointment]. IL TRATTAMENTO DEI CHELOIDI E DELLE CICATRICI IPERTROFICHE CON KELIMED.RTM. UNGUENTO." CHRONICA DERMATOLOGICA, Bd. 6, Nr. 2, 1996, Seiten 275-281, XP009000663
- "Contractubex-das Narbenspezificum" MERZ HOMEPAGE, [Online] XP002220461 Gefunden im Internet: URL:http://www.merz.de/wwwmerzde/patienten information/narben/contractubex__das_narbe nspezifikum.HTM> [gefunden am 2002-11-11]
- WILLITAL G.H. ET AL: "Efficacy of contractubex.RTM. gel in the treatment of fresh scars after thoracic surgery in children and adolescents." INTERNATIONAL JOURNAL OF CLINICAL PHARMACOLOGY RESEARCH, Bd. 14, Nr. 5-6, 1994, Seiten 193-202, XP009000661
- JACKSON B A ET AL: "Pilot study evaluating topical onion extract as treatment for postsurgical scars." DERMATOLOGIC SURGERY: OFFICIAL PUBLICATION FOR AMERICAN SOCIETY FOR DERMATOLOGIC SURGERY [ET AL.]. UNITED STATES APR 1999, Bd. 25, Nr. 4, April 1999 (1999-04), Seiten 267-269, XP001119165 ISSN: 1076-0512

## Beschreibung

Die vorliegende Erfindung betrifft ein neues fett(öl)haltiges Mittel, enthaltend Zwiebelextrakt, sowie seine Herstellung und seine Verwendung zur Pflege, Vorbeugung oder Behandlung von geschädigtem Hautgewebe, wie z.B. nach Operationen, Biopsien, Schnittwunden, Verbrennungen und sonstigen Unfällen, sowie insbesondere von Narben, Schwangerschaftsstreifen , degenerativen Hautveränderungen u.ä.. Das Mittel ist dadurch gekennzeichnet dass es eine Ölgrundlage aufweist und insofern z.B. auf Creme-, Lotion-, Fluid-, Massageöl-oder Balsamgrundlage hergestellt werden kann . Es ist daher fett(öl)haltig.. Überraschenderweise konnte der Zwiebelextrakt, welcher insbesondere Wasser, Alkohol, oder auch Wasser-Alkohol - haltig ist, in eine solche ölhaltige Grundlage eingebaut werden, ohne dass seine Wirksamkeit beeinträchtigt wird oder eine Phasentrennung auftritt. Die Wirksamkeit konnte in medizinischen Anwendungsbeobachtungen nachgewiesen werden.

Mittel zur Behandlung von geschädigtem Hautgewebe, insbesondere von Narben sind bekannt. Insbesondere werden hierfür gelartige Produkte eingesetzt. Die US - A - 5,885,581 beschreibt ein solches gelartiges Produkt, welches 20 - 30 Gewichtsprozent Polyethylenglykol 200, 0,005- 0,03 Gewichtsprozent Konservierungsmittel, 0,05- 0,2 Gewichtsprozent Sorbinsäure, 0,5- 2

Gewichtsprozent Allantoin, 1- 3 Gewichtsprozent Xanthan und wahlweise Parfümstoffe umfasst und welches gekennzeichnet ist durch 5- 15 Gewichtsprozent eines flüssigen Zwiebelextraktes (Extrakt Allium Cepa), auf der Basis eines wässrigen Trägers in einer Menge von etwa 55- 65 Gewichtsprozent. Das Produkt stellt somit ein fett(öl)- freies Gel dar und wird äußerlich auf geschädigtes Hautgewebe, insbesondere vemarbtes Gewebe aufgetragen. Das Produkt ist ferner gekennzeichnet durch einen pH- Wert von 4,5- 5,5 und eine Partikelgröße von weniger als 50µm.

Weiterhin ist ein Spezialpflaster gegen Narben bekannt, welches unter dem Namen "Hansaplast® Narbenreduktion" ohne Wirkstoffe zu einer besseren Heilung verletzter Haut führen soll. Dabei werden entsprechende Pflasterpads etwa 8- 10 Wochen auf die Haut gelegt und fördern durch das gesteigerte Temperaturniveau angeblich den Regenerationsprozeß.

Unter dem Produkt Kelofibrase® ist eine Narbencreme bekannt, welche als Wirkstoffe Harnstoff sowie Heparin- Natrium (60 000 I.E.) und Kampfer neben üblichen Öl/Emulgator Komponenten enthält. Dieses Produkt soll zur Narbenbehandlung, bei Narbenkontrakturen und bei Keloiden einsetzbar sein. Dabei wirkt Heparin- Natrium bekanntlich als blutverdünnendes Mittel.

Unter dem Namen Hirudoid®forte ist ein Gel bekannt, welches als Wirkstoff Mucopolysaccharidpolyschwefelsäureester (445mg, entsprechend 40 000 Einheiten in 100g Salbe) enthält. Weitere Inhaltsstoffe sind die für die Gelherstellung erforderlichen Komponenten wie Isopropylalkohol, Polyacrylsäure, Propylenglycol und Wasser. Mucopolysaccharidpolyschwefelsäureester haben im allgemeinen eine heparinoide Wirkung und entsprechen daher dem zuvor genannten Kelofibrase®-Produkt. Neben dem Gel ist auch eine Hirudoid®forte Salbe bekannt, die neben den oben bekannten Wirkstoffen ein Gemisch aus Mono- und Diglyceriden höherer Fettsäuren und mittelkettige Triglyceride etc. sowie Isopropylalkohol, Imid urea, Phenoxyethanol und Wasser aufweist. Derartige Produkte sind anwendbar zur Behandlung bei Phlebopathien, oberflächennahen Phlebitiden, Hämatomen und zur Auflockerung von harten Narben. Das Produkt darf nicht auf verletzte Haut aufgetragen werden.

Unter dem Namen Hylaform® ist ein Gelimplantat bekannt, welches quervernetzte Hyaluronsäure enthält, die in einer wässrigen kochsalzhaltigen Lösung zwecks Injektion vorhanden ist. Mit einem solchen Mittel sollen Hautdeformationen behandelbar sein. Akute oder chronische Hautkrankheiten im betroffenen Korrekturbereich dürfen jedoch nicht vorliegen.

Unter dem Namen Linoladiol® N ist eine hydrophile O/W- Creme bekannt, welche in der Cremegrundlage als Wirkstoff Estradiol enthält. Mittels dieses hormonhaltigen Präparates sind neben gynäkologischen Applikationen auch dermatologische Anwendungen, wie Verbrennungen, Narbennachbehandlung, Hautatrophie, periorale Dermatitis und Ekzeme im akuten und subakuten Stadium angegeben. Hierbei sind allerdings die für hormonhaltige Produkte bekannten Anwendungbeschränkungen wie Nebenwirkungen bzw. Wechselwirkungen erheblich zu beachten.

Unter dem Produktnamen PC 30 V ist ein Liquidum beschrieben, welches Roßkastaniensamentrockenextrakt sowie Kamillenblütentrockenextrakt in 1,3-Butandiol, Dexpanthenol, Allantoin und Geruchsstoffe enthält. Dieses Mittel soll einsetzbar sein bei der Behandlung von Hautschädigungen wie Wundscheuern empfindlicher Druckstellen und Narben durch orthopädische Apparate, sowie Wundliegen. Eine Indikation bezüglich Narben durch Operationen oder sonstige Hautschädigungen ist hier nicht angegeben.

Die Salbe Striatridin® enthält neben der Salbengrundlage als Wirkstoff alkylverzweigte Fettsäureester des Octadecylalkohols, Aminosäuresol sowie Ethylnicotinat. Dieses Produkt ist zur Narbenbehandlung, bei Schwangerschaftsstreifen sowie bei schlaffer, funktionsgeschädigter Haut einsetzbar.

Die DE- A 196 28 284 beschreibt den Einsatz von Bärlauch zur Behandlung von Schuppenflechte.

Die DE- A 37 23 248 betrifft die Verwendung von Thiosulfinsäurederivaten zur Behandlung von Entzündungen. Diese können u.a. durch Extraktion von Zwiebeln und nachfolgende Chromatographie gewonnen werden. Zwiebelextrakt selbst wird hier nicht eingesetzt.

EP- B 429 080 betrifft ein Herstellungsverfahren für S- Allylcystein enthaltende Produkte, wobei z. B. wässrige Knoblauch -Extrakte mit Cystein versetzt werden, wodurch S-Allylcystein entsteht.

EP- B 364 442 betrifft einen Ölextrakt aus mindestens 3 verschieden Kräutern, ausgewählt aus Wolfsmilch, Veronica, Schafgarbe, Erdrauch, Knoblauch, Nessel und Ringelblume. Diese Kombination wird als Öl, z.B. mit Paraffin gegen Schuppenflechte angewendet.

EP-B EP 201 956 betrifft die Extraktion und chromatographische Fraktionierung z.B. von Tabak, Algen, Knoblauch, wobei die erhaltenen spezifischen Substanzen als antioxidative Substanzen in Kosmetika eingesetzt werden sollen.

US- A 6,200,570 betrifft Zusammensetzungen, enthaltend Knoblauchextrakt und mindestens einen weiteren Pflanzenextrakt wie Aloe Vera sowie entzündungshemmende Mittel wie Diclofenac mit anti- allergischer, analgesischer Wirkung.

JP-A 2000327535 beschreibt ein Haartonikum, welches z.B. Allium Schoenoprasum und/oder andere Pflanzenextrakte aufweist.

JP-A 09194334 betrifft ein gegen Haarausfall wirksames Haartonikum, das z. B. Allium Sativum und/oder andere Pflanzenextrakte aufweist.

JP-A 08012570 und JP-A 0317413 betreffen Pflanzenextrakte wie Allium sativum oder Allium victorialis enthaltende anti- allergene oder Anti- Schuppenmittel.

Aus dem vorgenannten Stand der Technik ergibt sich, dass die zur Narbenbehandlung einsetzbaren Produkte entweder als Gel vorliegen oder, wenn sie nicht als Gel vorliegen, Wirkstoffe enthalten, welche erhebliche Nebenwirkungen mit sich bringen können. So ist z.B. das Estradiol enthaltende Produkt anwendungsbeschränkt im Hinblick auf den Hormongehalt. Mucopolysaccharidpolyschwefelsäureester mit ihrer heparinoiden Wirkung können Überempfindlichkeitsreaktionen auslösen und dürfen auf keinen Fall auf eine verletzte Haut aufgetragen werden. Das oben genannte Hylaform®-Hyaluronsäure enthaltende Produkt ist ein Implantat, welches nur zur mechanischen Korrektur von Hautdeformationen geeignet sein kann und insofern keine dauerhafte Einwirkung auf das Hautgewebe selbst mit sich bringt. Das Liquidum PC 30 V ist lediglich zur Druckstellennarbenbehandlung geringen Umfangs, wie z.B. durch orthopädische Apparate verursacht, einsetzbar und insofern zur dauerhaften Veränderung geschädigten Hautgewebes durch Operationen, Verletzungen, Verbrennungen etc. nicht beschrieben.

Es besteht daher ein Bedürfnis nach einem Produkt, welches die oben genannten Nebenwirkungen nicht aufweist. Ferner sollte es nicht in Gelform vorliegen, da fett(öl)- freie Gele bekanntlich eine eher austrocknende Wirkung auf die Haut ausüben können.

Aufgabe vorliegender Erfindung ist es daher, ein Produkt bereitzustellen, welches einen gut verträglichen Wirkstoff aufweist und wobei dieser in eine Emulsionsgrundlage eingearbeitet ist, wodurch die Haut nicht ausgetrocknet wird und darüber hinaus noch ein positiver, pflegender, insbesondere elastizitätsfördernder Hautpflegeeffekt erzielt werden kann.

Diese Aufgabe wird erfindungsgemäß gelöst durch ein Mittel, welches als Wirkstoff einen Zwiebelextrakt aufweist und neben den üblichen Zusatzstoffen, ausgewählt aus Konsistenzgebern, Farbstoffen, Antioxidantien, Parfümstoffen, Feuchthaltemittel, Konservierungsmittel, Stabilisatoren, Zusatzwirkstoffen, eine Fett(Öl-)phase aufweist.

Bevorzugt können zusätzlich eine Wasserphase und entsprechende Emulgatoren, ausgewählt aus der Gruppe aus O/W- und/oder W/O Emulgatoren oder Mischungen hiervon oder mit geeigneten Co-Emulgatoren enthalten sein.

Die Mittel können insgesamt 5-99% Fett(öl)phase, 0,1-35% Zusatzstoffe, 1-20% Zwiebelextrakt und die übrigen Stoffe, sofern vorhanden, in den unten angegebenen Mengen aufweisen.

Besonders geeignet sind Mittel, welche zusammengesetzt sind aus 70-99, ganz besonders 80-98% Fett(öl)phase, 0,1-20%, insbesondere 0,1-10%, Zusatzstoffe und 1-20%, bevorzugt 1-10% und ganz besonders 2-5%, Zwiebelextrakt enthalten, wobei bevorzugt als Rest Lösungsmittel vorliegen können, wie z. B. Alkohole (Ethanol, Isopropanol) z.B. in Mengen von bis zu 20%.

Das erfindungsgemäße Mittel ist ferner bevorzugt zusammengesetzt aus einer Fett(öl)phase in einer Menge von 5- 70%, 0,1- 15% Emulgator (O/W; W/O); Mischungen hiervon oder mit Co-Emulgatoren, 0,1- 35% Zusatzstoffen, 1- 20% Zwiebelextrakt und der Rest Wasser oder ein Wasser/Alkohol- z. B. Ethanol, Isopropanol, -Mischungen, z.B. bis 20% .

Insbesondere sind als Fett(öl) phase 5-60%, bevorzugt 5-40% und ganz besonders 5-25% geeignet.

Der Zwiebelextrakt ist insbesondere ein Wasser - oder Alkohol - oder Wasser-Alkohol - aufweisender Zwiebelextrakt.

Mit einer solchen Zusammensetzung ist es überraschenderweise möglich, den Zwiebelextrakt so in eine Fett(öl)haltige Phase einzuarbeiten, dass hierbei eine gleichförmige Verteilung des Extraktes stabil vorliegt und darüber hinaus mittels dieser Grundlage ein Austrocknen der behandelten Haut nicht stattfinden kann und somit auch ein pflegender, elastizitätsfördernder Effekt erzielt wird, so dass die Behandlung der geschädigten Hautteile sowohl hinsichtlich der Vernarbung bzw. Erschlaffung als auch bezüglich der Hautweichheit positiv beeinflusst bzw. verhindert werden kann.

Besonders geeignet sind im erfindungsgemäßen Mittel zwischen 1- 15%, 2-15%, insbesondere 5-15% des Zwiebelextraktes, bezogen auf die Gesamtmenge , vorzugsweise 5- 10, insbesondere 8- 10% und ganz besonders bevorzugt 2-4% oder 10%.

Die Zusatzstoffe liegen bevorzugt bei 0,1- 30, insbesondere 0,1-25% je nach Anwendungszweck vor.

Die Menge an Emulgatoren beträgt insbesondere 0,1-10%,insbesondere 1-10%, bevorzugt 1-8% und ganz besonders 1-5%.

Der Zwiebelextrakt ist insbesondere ein wässrig - ethanolischer Extrakt . Er weist bevorzugt Wasser und 10-15% Ethanol auf.

Es kann auch von Vorteil sein, einen alkoholischen Zwiebelextrakt einzusetzen. Dieser kann neben Alkohol vorzugsweise auch 10-80%, insbesondere 20-60% eines Lösungsmittels, ausgewählt aus Triglyceriden, Kohlenwasserstoffen und Fettsäureestern aufweisen.

Die Mengenangaben beziehen sich auf Gewichts %, sofern nicht anders angegeben.

Die Fett(öl)phase ist bevorzugt ausgewählt aus Kohlenwasserstoffen, Fettalkoholen,-ethern und -estern, (Poly)olfettsäureestern, Triglyceriden, natürlichen Ölen, natürlichen Fetten, Wachsen, Silikonölen, Silikonwachsen oder Mischungen hiervon. Insbesondere sind hier flüssige Paraffine, Milchsäureester, Fettalkoholether, Nachtkerzenöl, Silikonöl oder Mischungen hiervon bevorzugt.

Der W/O-Emulgator weist vorteilhafter weise einen HLB-Wert von 1-9, insbesondere 1-8, bevorzugt 2-7 und ganz besonders bevorzugt 3-6 und der O/W- Emulgator einen HLB- Wert von 9-18, bevorzugt 9-15 und insbesondere 9-13, auf oder ist ein ionischer O/W- Emulgator.

Als W/O-Emulgatoren sind insbesondere bevorzugt Sorbitan- Derivate, Polyethoxylierte Fettsäuren/Alkohole/Ester/,Triglyceride, (Poly)glycerylderivate, Polyolester, Glucose-Derivate, Pentaerythrit-Derivate, Alkylphenole, (Block)Polymere, Fettsäuresalze, Siloxane oder Mischungen hiervon und hierunter ganz besonders Abil® EM 90, Arlacel ®582 und Magnesiumstearat oder Mischungen hiervon aufweist.

Es können hier auch Co- Emulgatoren vorhanden sein, wie z.B. Arlatone®T(V).

Als O/W-Emulgator eignen sich insbesondere polyoxyethylierte Produkte, nichtionische und ionische Phosphate, ionische einwertige Salze, (Poly)glycerylester, Zuckerester, Sterol-Derivate, Rizinusöl-Derivate, Siloxane oder Gemische hiervon oder Mischungen mit Co-Emulgatoren hiervon. Insbesondere sind hier Tego Care® 450, Eumulgin ®B1 oder Mischungen hiervon und/oder mit Co-Emulgatoren geeignet.

Ganz besonders bevorzugt sind bei Anwesenheit von O/W-Emulgatoren Stabilisatoren, ausgewählt aus Acrylamiden, Acrylaten und Polysacchariden, insbesondere solche, wie sie nachfolgend beschrieben werden.

Es ist femer bevorzugt, wenn die erfindungsgemäßen Mittel weiterhin als Zusatzstoffe, solche ausgewählt aus Vitaminen, Elektrolyte, wie z. B. Magnesiumsulfat oder Natriumchlorid, Allantoin, D- Panthenol, Hyaluronsäure, Mucopolysacchariden, Farbstoffen, Parfümstoffen, Konservierungsmittel, Feuchthalter aufweisen.

Insbesondere können auch zusätzlich Wachsprodukte sowie oder alternativ als Zusatzstoff weiterhin Lecitine, insbesondere Phosal®50 SA enthalten sein.

Das Mittel kann unterschiedliche Mengen an Wasser, Fettphase, Emulgator, Zusatzstoffe und Wirkstoff (Zwiebelextrakt) enthalten. Hierbei werden dann je nach Anwendungszweck z.B. eine Lotion, ein Fluid, eine Creme oder ein Balsam/Salbe erhalten.

Das Mittel kann somit bevorzugt zusammengesetzt sein aus einer Fett(öl)phase in einer Menge von 5-70% Gewichtsprozent, , insbesondere 5-55%, einem oder mehreren W/O-Emulgatoren, Mischungen hiervon oder mit Co-Emulgatoren in einer Gesamtmenge von 0,5- 15%, insbesondere 0,5-10%, sowie 0,1- 25 %, insbesondere 0,1-22%, an Zusatzstoffen der oben genannten Art, sowie 1- 15, insbesondere 1-10% des oben genannten Zwiebelextraktes und als Rest Wasser oder ein Gemisch von Wasser mit Alkoholen wie z.B. Ethanol , Isopropanol in Mengen bis zu 20%.

Das Mittel kann insbesondere auch zusammengesetzt sein aus 5- 40%, insbesondere 5-30%, Fett(öl)phase, 0,1- 15%, insbesondere 1-10% eines oder mehrerer O/W- Emulgatoren oder Mischungen hiervon oder mit Co-Emulgatoren, 0,1- 32%, insbesondere 0,1-20% Zusatzstoffe und 1- 10% Zwiebelextrakt und als Rest Wasser oder ein Gemisch von Wasser mit Alkoholen wie z.B. Ethanol , Isopropanol in Mengen bis zu 20%.

Auf diese Weise kann das Mittel zur Behandlung von Narben bzw. von geschädigtem und/oder erschlafftem Hautgewebe insbesondere als z.B. Massageöl oder Balsam, Lotion, Fluid oder Creme hergestellt werden. So können bei Verwendung von W/O- Emulgatoren insbesondere Lotionen, und ein Balsam und bei Einsatz von O/W- oder W/O- Emulgatoren insbesondere cremeartige Produkte oder Fluids entstehen. Je nach Einsatzgebiet kann zwischen diesen Produkten ausgewählt werden. Insbesondere geeignet ist eine O/W-Creme der oben beschriebenen Art zur Behandlung z.B. von Aknenarben. Sollen größerflächige Bereiche behandelt werden, empfiehlt sich eine Lotion, die bevorzugt bei der Behandlung oder auch zur vorbeugenden Verhinderung von Schwangerschaftsstreifen oder allgemein erschlaffter Haut einsetzbar ist. Ebenfalls größerflächig einsetzbar ist ein Balsam, der ganz besonders zur Behandlung von Operations- oder Verbrennungsnarben, Schnittwunden geeignet ist. Massageöl ist insbesondere geeignet zur Vorbeugung oder Pflege und Behandlung von größeren Hautflächen wie z.B. bei Schwangerschaftssteifen.

Die Darreichungsform ist jedoch nicht auf eine bestimmte Indikation beschränkt und kann varriert werden.

Die einzelnen Inhaltsstoffe werden nachfolgend näher beschrieben:

### I. Zwiebelextrakt

Als Zwiebelextrakt eignet sich insbesondere ein Wasser , Alkohol oder Wasser-Alkohol enthaltender Auszug aus getrockneten Zwiebeln. Die Extraktion kann dabei mit Wasser selbst oder auch mit Alkohol, ggf. unter Zusatz von Lösungsmitteln wie nachstehend genannt, oder einem Gemisch aus Wasser und einem oder mehreren Alkoholen, wie nachfolgend genannt, vorgenommen werden. Die getrockneten Zwiebeln, auch als getrocknete Zwiebelchips bekannt, erhalten aus der Stammpflanze Allium cepa Linne, können dabei zunächst mit dem Extraktionsmittel extrahiert werden, indem man die Droge vorzugsweise in der Wärme, z.B. bei 40-90°C, erschöpfend perkoliert. Das Perkolat kann dann bei geeigneter, insbesondere erhöhter Temperatur über 30°C unter Vakuum zum Spissum- Extrakt (eingedickter Extrakt) eingedampft und anschließend im gewünschten Lösungsmittel zum Wasser-, Alkohol- oder Wasser-Alkoholgemisch - Fluidextrakt aufgelöst werden. Alternativ kann der Fluidextrakt auch ohne zuvoriges Eindampfen durch direktes Umsetzen mit dem gewünschten Lösungsmittel erhalten werden.

Erhalten wird eine rötlich- braune bis braune Flüssigkeit von charakteristischem Geruch. Das Verhältnis von trockener Droge zu Fluidextrakt kann dabei variieren von 0,1:1 bis 10:1, bevorzugt 0,1:1 bis 5:1, insbesondere 0,15:1 bis 4:1. Ganz besonders bevorzugte Verhältnisse sind 0,16:1 oder 4:1.

Besonders bevorzugt sind Fluidextrakte der genannten Art mit Wasser/Alkohol-Gemisch oder nur Alkohol als Lösungsmittel. Im Gemisch ist bevorzugt 5-60%, insbesondere 10-50% und ganz besonders 10-40% oder auch 10-15, vor allem 13%, Alkohol, bevorzugt Ethanol, enthalten.

Der Alkohol ist vorzugsweise ausgewählt aus Ethanol, Isopropanol oder Propanol oder zweiwertigen Alkoholen wie Butylenglykol, Propylenglykol oder Mischungen hiervon. Insbesondere bevorzugt ist Ethanol.

Es kann femer von Vorteil sein, wenn der Zwiebelextrakt, welcher mit Wasser oder anderen wie z.B. den oben genannten alkoholischen Lösungsmitteln extrahiert wird, zum alkoholischen Fluidextrakt wie beschrieben, aufgearbeitet wird. Insbesondere können hierbei 10-80%, bevorzugt 20-60% des Alkohols durch ein Lösungsmittel, ausgewählt aus den nachfolgend unter Punkt II genannten Stoffen, insbesondere Kohlenwasserstoffe, Triglyceride, besonders mittelkettige, und Fettsäureester oder Mischungen hiervon, ersetzt werden.

Ein erfindungsgemäß verwendbarer Zwiebelextrakt kann beispielsweise wie folgt hergestellt werden:
Die getrockneten Zwiebeln, auch Zwiebelchips genannt, können z.B. mit gereinigtem Wasser z.B. im Verhältnis von Droge zu Auszugsmittel (Wasser) von 1:16 extrahiert werden. Das Verhältnis von trockener Droge zu nativem Extrakt entspricht dann 1,8:1, bzw. 1,5-2,2:1. Das Verhältnis von trockener Droge zu Fluidextrakt ist dann 0,16:1. Die Droge (z.B. 16kg Chips) wird mit heißem Wasser (80- 90 Grad Celsius) erschöpfend perkoliert. Das Perkolat kann dann bei ca. 55 Grad Celsius unter Vakuum zum Spissum-Extrakt (eingedickter Extrakt) eingedampft werden. Dabei wird kurzzeit erhitzt (z.B. 3 Sekunden bei 141 Grad Celsius). Der erhaltene Spissumextrakt ist dickflüssig und wird in Wasser, Alkohol oder im Wasser- Alkoholgemisch zum Fluidextrakt z.B. o.g. Verhältnis aufgelöst. Erhalten wird eine rötlich- braune bis braune Flüssigkeit von charakteristischem Geruch. Dieser Extrakt ist mit Wasser in jedem Verhältnis mischbar, weist eine relative Dichte bei 20 Grad Celsius von 1,00- 1,03 g/ml auf. Sofern mit Wasser/Alkohol, insbesondere Ethanol gearbeitet wurde, können z.B. etwa 13- 20% (V/V) Alkohol (z.B. Ethanol) vorliegen. Der Trockenrückstand (nach 2 Stunden bei 105 Grad Celsius) beträgt mindestens 7,0 % (m/m). Hinsichtlich der mikrobio-logischen Reinheit entspricht das Produkt den Anforderungen der Kategorie 3 des DAB 10 .
Die charakteristischen Inhaltsstoffe der Zwiebeln sind Derivate von schwefelhaltigen Aminosäuren . Daneben liegen noch Eiweiß, Fett und Kohlenhydrate vor.

Das wie oben beschrieben hergestellte Produkt ist beispielsweise erhältlich von Fa. Finzelberg Extrakte und beschrieben in der o. g. US-A-5885581.

Dieser wie oben beschrieben gewonnene Extrakt ist im Narbengel, gemäß der US Patentschrift 5885581 eingesetzt. Im Unterschied zur Gelzusammensetzung war es jedoch überraschend, diesen Extrakt in eine ölhaltige oder Öl-Wasserhaltige Grundlage einzuarbeiten, wobei gleichzeitig eine vollständige und homogene Verteilung der Wirkstoffe und auch insbesondere ihr Intaktbleiben gewährleistet bleibt, zumal, wenn der Anteil an dieser Extraktphase, insbesondere in wässriger, alkoholischer, wässrig/alkoholischer Form, sehr hoch ist.

Bevorzugt wird also ein Wasser- Alkohol- Zwiebelextrakt in den erfindungsgemäßen Mitteln eingesetzt, insbesondere ein wie oben beschrieben Wasser-Ethanol Extrakt, vorzugsweise mit 10-40, insbesondere 13-20%, insbesondere 10-15% Ethanolanteil.

Wie erwähnt kann der Zwiebelextrakt statt Ethanol auch andere Lösungsmittel, insbesondere Glykol, Butylenglykol, Propylenglykol, aufweisen.

Ferner bevorzugt ist auch ein Alkohol-, bevorzugt -Ethanol- haltiger Extrakt, welcher insbesondere darüber hinaus wie erwähnt , noch weitere Lösungsmittel, z.B. 10-80%, insbesondere 20-60%, wie die unter Punkt II genannten aufweisen kann. Bevorzugt sind hier Kohlenwasserstoffe, Triglyceride z. B. mittelkettige, Fettsäureester.

Ferner sind Mischungen aus einem Wasser-Alkohol, insbesondere Ethanol-Zwiebelextrakt, z.B. mit 10-40% Ethanol wie oben beschrieben und einem alkoholischen, insbesondere ethanolischen Zwiebelextrakt mit 10-80%, insbesondere 20-60%,Lösungsmittel wie die unter Punkt II genannten , insbesondere hier Kohlenwasserstoffe, Triglyceride z. B. mittelkettige, Fettsäureester geeignet. Das Verhältnis von Wasser/Alkohol- und Alkohol/Lösungsmittel-Zwiebelextrakt kann insbesondere 3:1 bis 1:3, bevorzugt 1:1 betragen.

### II.Fett(Öl)phase

Für die Fett(Öl-)phase können hierfür übliche Öl- Komponenten eingesetzt werden. Hierzu gehören:
Übliche, vorzugsweise flüssige Lipide, diese können einzeln oder im Gemisch vorliegen.
Insbesondere sind die folgenden Gruppen und Beispiele hierfür geeignet:
   1.Kohlenwasserstoffe
      Kohlenwasserstoffe wie Squalen, Squalan, insbesondere auch flüssige Paraffine (Paraffinum Perliquidum), Isoparaffine, Dioctylcyclohexane (Cetiol® S), Isohexadecane (Arlamol® HD);
   2.Fettalkohole wie Oleylalkohol, Octyldodecanol (Eutanol® G);
   3.Fettsäureester, z.B. Isopropylfettsäureester (Palmitat, Myristat, Isostearat, Oleat), Decyl Oleate (Cetiol® V), Hexyl Laurate, C 12 - 15 Alkyl Benzoate (Finsolv® TN), Dicaprylyl Carbonate (Cetiol® CC), Diester wie Dibutyladipate (Cetiol®B), Propylenglykol Dipelargonate, verzweigte Fettsäureester wie PCLliquid® (Cetearyl Octanoate) oder Gemische wie Cetiol® PGL (Hexyldecanol und Hexyldecyl Laurate);
   4.Fettalkoholether wie Dicaprylyl Ether (Cetiol® OE) oder Fettalkoholester wie Milchsäurester wie C12-13 Alkyl Lactate (Cosmacol® ELI);
   5.Polyolfettsäureester wie Cetiol® HE (PEG-7 Glyceryl Cocoate);
   6.Trictlyceride, insbesondere mittelkettige (Neutralöle) wie Caprylic/Capric Triglyceride (Miglyol® 810, 812) sowie deren Polyolester wie Propylene Glycol Dicaprylate/ Dicaprate (Miglyol® 840);
   7.Natürliche Fette und Öle wie Sonnenblumen-, Soja-, Pfirsichkern-, Aprikosenkern-, Traubenkern-, Ricinus-, Erdnuß-, Mandel-, Nerz-, Weizenkeim-, Avocadoöl, Nachtkerzenöl;
   8.Wachse, wie natürliche flüssige Wachse z.B. Jojobaöl oder dessen Substitut Oleyl Erucate (Cetiol® J 600) oder synthetische Wachse wie die nachfolgend unter "Konsistenzgeber" beschriebenen.
   9.Silikonöle und -wachse, z.B. Polydimethylsiloxane wie Dow Corning Fluid® 200 (Dimethicone), Cyclomethylsiloxane wie Dow Corning Fluid® 345 (Cyclomethicone), Phenylmethylpolysiloxane wie Phenyl Dimethicone (Abil® AV 8853) oder Alkyl-Polymethylsiloxan-Copolymere wie Cetyl Dimethicone (Abil® Wax 9801), Stearyl Dimethicone (Abil® Wax 9800), Dialkoxydimethylpolysiloxane wie Stearoxy
      Dimethicone (Abil® Wax 2434), Behenoxy Dimethicone (Abil® Wax 2440)

Besonders bevorzugte Öl-Komponenten sind flüssige Paraffine, Fettsäureester wie Isopropylpalmitat oder -myristat, Fettalkoholether wie Dicaprylyl Ether (Cetiol®OE) sowie die genannten natürlichen Fette und Öle, insbesondere Avokado-, Soja-, Pfirsichkern-, Aprikosenkernöl und ganz besonders Nachtkerzenöl, insbesondere Mischungen hiervon, Silikonöle der vorbeschriebenen Art sowie Milchsäureester z.B. Cosmacol® ELI sowie Mischungen hiervon mit den vorgenannten Komponenten.

Insbesondere bevorzugt werden die genannten flüssigen Paraffine, Milchsäureester, Fettalkoholether, Nachtkerzenöl und Silikonöle, auch in Kombination miteinander, wobei ca. 1- 50% an Einzelkomponente bezogen auf die Gesamtmenge an Öl vorhanden sein können.

Ferner sind auch Silikonwachse besonders geeignet, vor allem auch Kombinationen hiervon und auch mit den vorgenannten Paraffinen, Milchsäureester und Nachtkerzenöl.

### III.Emulgatoren

### A). W/O Emulgatoren

Als W/O- Emulgator/en eignen sich solche mit einem geeigneten HLB- Wert von 1- 9, insbesondere1- 8. Besonders bevorzugt sind folgende Produkte:
1.Sorbitan-Derivate
   Sorbitan Ester wie Sorbitan Oleate (Span® 80, HLB = 4,5), Sorbitan Stearate (HLB = 5,0), Sorbitan Sesquioleate (Crill® 43, HLB = 3,7), Sorbitan Isostearate (Crill® 6, HLB = 4,7), Sorbitan Tristearate (Crill® 35, HLB = 2,1);
   sowie Arlacel® 582 (Sorbitan Isostearate, PEG-2 Hydrogenated Castor Oil, Ozokerite, Hydrogenated Castor Oil, HLB = 5).
2.Polyethoxylierte Produkte
   Polyethoxylierte Fettsäuren und -alkohole wie PEG-2 Oleate (HLB = 5,0), PEG-4 -Distearate (HLB = 3,0), PEG-2 Stearate (HLB = 4,4), Ceteareth-3, (Volpo® CS3, HLB = 5,0), Ceteth-2 (Volpo® C2, HLB = 5,3);
   Polyoxyethylen Fettsäureester wie Arlatone® T(V) (PEG-40 Sorbitan Peroleate, HLB = 9);
   Ethoxylierte Triglyceride wie PEG-5 Castor Oil (HLB = 3,9), PEG-6 Diricinoleate (HLB = 5,0), PEG-7 Hydrogenated Castor Oil (Cremophor® WO 7, HLB = 5,0);
3.(Poly)glyceryldehyate
   Polyglycerylester wie Polyglyceryl-3 Diisostearate (Lameform® TGI, HLB = 3,5), Polyglyceryl-2 Dipolyhydroxystearate (Dehymuls® PGPH, HLB = 3,5), Diisostearoyl Polyglyceryl-3 Diisostearate (Isolan® PDI, HLB ca. 5), Polyglyceryl-3 Oleate (Isolan® GO 33, HLB ca. 5), Polyglyceryl-3 Dioleate (Cremophor® GO 32, HLB ca. 5), Polyglyceryl-4 Isostearate (Isolan® GI 34, HLB ca. 5);
   Glycerin-Ester wie Glyceryl Ricinoleate (Cithrol® GMR N/E, HLB = 2,7), Glyceryl Laurate (Cithrol® GML N/E, HLB = 4,9), Glyceryl Dioleate S/E (Cithrol® GDO S/E, HLB = 2,9).
4.Polyolester
   Polyol-Ester wie Glycol Oleate S/E (Cithrol® EGMO S/E, HLB = 2,7), Glycol Ricinoleate (Cithrol® EGMR S/E, HLB = 2,0), Glycol Dilaurate S/E (Cithrol® EGDL S/E, HLB = 2,0), Propylene Glycol Ricinoleate (Cithrol® PG MR S/E, HLB = 3,6), Propylenglykol Laurate (Cithrol® PGML N/E, HLB = 2,7);
5.Glucose-Derivate
   Glucose Ester wie Methyl Glucose Dioleate (Isolan® DO, HLB ca. 5), Methyl Glucose Isostearate (Isolan® IS, HLB ca. 5);
6.Pentaerythrit-Derivate
   Pentaerythrit-Fettsäureester, z.B. Pentraerythrityl Monolaurate (HLB = 4,8), Pentaerythrityl Monotallate (HLB = 4,0) oder Mischester, z.B. mit Citronensäure Fettalkoholester wie Dehymuls® E (Dicocoyl Pentaerythrityl Distearyl Citrate, Sorbitan Sesquioleate, Cera Alba, Aluminium Stearate, HLB = 4,0), Dehymuls® F (Dicocoyl Pentaerythrityl Distearyl Citrate, Cera Microcristallina, Glyceryl Oleate, Aluminium Stearate, Propylene Glycol, HLB = 4,0);
7.Alkylphenole
   z.B. Nonoxynol-2 (HLB ca. 4,5);
8.Polymere
   Polymere wie Polyoxypropylen-polyoxyethylen-Blockpolymere (INCI-Name: Poloxamere), z.B. Pluronic® PE 3100 (HLB = 4,5), Pluronic®PE 6100 (HLB = 3,0) oder PEG-30 Dipolyhydroxystearate (Arlacel® P 135, HLB ca. 5,5);
9.Siloxan-Derivate
   Polysiloxan-Copolymere wie Polysiloxan-Polyether-Copolymere, insbesondere Polysiloxan-Polyalkyl-Polyether-Copolymere wie Cetyl Dimethicone Copolyol (Abil® EM 90, HLB = ca. 5), Laurylmethicone Copolyol (Dow Corning® Q2-5200, HLB ca. 4) oder deren Gemische wie Abil® WE 09 (Cetyl Dimethicone Copolyol Polyglyceryl-4-Isostearate, Hexyl Laurate, HLB ca. 5).
10.Fettsäure-Salze
   mehrwertige Salze wie Magnesium-, Aluminium-, oder Zinkstearat, wobei Magnesiumstearat bevorzugt ist.

Insbesondere bevorzugte Emulgatoren sind Abil ® EM 90 (Cetyl Dimethicone Copolyol) oder Adacel® 582 (Sorbitan Isostearate, PEG-2 Hydrogenated Castor Oil, Ozokerite, Hydrogenated Castor Oil, HLB = 5) oder Magnesium-, Aluminium-,Zink-stearat, insbesondere Mischungen hiervon. Ferner können die genannten Emulgatoren auch mit geeigneten Co-Emulgatoren kombiniert werden, so dass der gewünschte HLB- Wert vorliegt. Hierzu gehört z.B. Arlatone® T(V), insbesondere in Kombination mit den bevorzugten W/O-Emulgatoren.

### B) O/W Emulgatoren

Als O/W Emulgatoren sind insbesondere solche mit einem HLB- Wert von 9- 18, bevorzugt 9- 15 und insbesondere 9- 13 geeignet.

Hierzu gehören bevorzugt polyoxyethylierte Produkte wie (HLB- Werte rechts):

| | | |
|---|---|---|
| G - 2111 | Polyoxyethylen- oxypropylenoleat | 9,0 |
| G - 2125 | Tetrathylenglykolmonolaurat | 9,4 |
| Brij® 30 | Polyoxyethylenlaurylether | 9,5 |
| Tween® 61 | Polyoxyethylensorbitanmonostearat | 9,6 |
| Tween® 81 | Polyoxyethylensorbitanmonooleat | 10,0 |
| G - 3806 | Polyoxyethylencetylether | 10,3 |
| Tween® 65 | Polyoxyethylensorbitantristearat | 10,5 |
| Tween® 85 | Polyoxyethylensorbitantrioleat | 11,0 |
| G - 3910 | Polyoxyethylenoleyether | 12,2 |
| G - 2127 | Polyoxyethylenmonolaurat | 12,8 |
| Renex® 690 | Polyoxyethylenalkylarylether | 13,0 |
| | Polyethylenglykol-400-monolaurat | 13,1 |
| Cremophor® EL | Polyoxyethylen- Rizinusöl | 13,3 |
| G - 1284 | Polyoxyethylen- Rizinusöl | 13,3 |
| Tween® 21 | Polyoxyethylensorbitanmonolaurat | 13,3 |
| Renex® 20 | Polyoxyethylenester gemischter Fett' und Harzsäuren | 13,5 |
| G - 1441 | Polyoxyethylensorbit - Lanolinderivat | 14,0 |
| G - 7596J | Polyoxyethylensorbitanmonolaurat | 14,9 |
| Tween® 60 | Polyoxyethylensorbitanmonostearat | 14,9 |
| Tween® 80 | Polyoxyethylensorbitanmonooleat | 15,0 |
| Myrj® 49 | Polyoxyethylensorbitanmonostearat | 15,0 |
| G - 3720 | Polyoxyethylenstearylether | 15,3 |
| G - 3920 | Polyoxyethylenoleyether | 15,3 |
| Tween® 40 | Polyoxyethylensorbitanmonopalmitat | 15,6 |
| G - 2162 | Polyoxyethylen oxypropylenmonostearat | 15,7 |
| Cremophor® 0 | Polyoxyethylenfettalkoholether | 16,0 |
| G - 1471 | Polyoxyethylensorbit - Lanolinderivat | 16,0 |
| Myrj® 51 | Polyoxyethylenmonostearat | 16,0 |
| Cetomacrogol 1000 | Polyoxyethylenglykol-1000-Monocetylether | 16,1 |
| Tween® 20 | Polyoxyethylensorbitanmonolaurat | 16,7 |
| Brji® 35 | Polyoxyethylenlaurylether | 16,9 |
| Myrj® 52 | Polyoxyethylenmonostearat | 16,9 |
| Myrj® 53 | Polyoxyethylenmonostearat | 17,9 |

Ferner sind bevorzugt auch:

| | | |
|---|---|---|
| Myrj® 45 | Polyoxyethylenglykolmonostearat | 11,2 |
| | Polyoxyethylenglykol-400-monooleat | 11,4 |
| Cremophor® AP- fest | Polyoxyethylenglykol- 400-monostearat | 11,6 |
| G - 2161 | Polyoxyethylenglykol400-monostearat | 11,6 |
| Brij® 721 P | Steareth-21 | 15,5 |
| Eumulgin®B1 | Ceteareth-12 | 13 |

Bevorzugt sind auch handelsübliche Gemische mit Co-Emulgatoren wie Fettalkoholen oder Glycerylestern wie Emulgator E 2149 (Steareth-7, Stearyl Alcohol, HLB = 11) oder Arlacel® 165 FL (Glyceryl Stearate, PEG-100 Stearate, HLB = 11)
oder auch

| | | |
|---|---|---|
| Atlox® 3300 | Alkylarylsulfonattriethanolamioleat | 11,7 |

Weiterhin bevorzugt sind nichtionische und ionische Phosphate wie Cetyl- oder Stearylphosphate (HLB = 8, bzw. 10), Trilaureth-4 Phosphate (Hostaphat® KL 340, HLB ca. 13), Triceteareth-4 Phosphate (Hostaphat® KW 340, HLB = 10), (Poly)glycerylester wie Dermofeel®Q 182 S (Polyglyceryl-10-Distearat) oder PEG-4 Polyglyceryl-2 Stearate (Hostacerin® DGSB, HLB ca. 8), Polyglyceryl-2 PEG-10 Laurate (Hostacerin® DGL, HLB ca. 14), Polyglyceryl-3 Methyl Glucose Distearate (Tego Care® 450, HLB = 12) oder Gemische wie Eumulgin® VL 75 (Lauryl Glucoside, Polyglyceryl-2 Dipolyhydroxystearate, Glycerin, HLB ca. 13). Ferner bevorzugt sind Zuckerester wie Glucoseester z.B. Cetearyl Glucoside (Tego Care® CG 90, HLB = 11), Methyl Glucose Sesquistearate (Tego Care® PS, HLB = 12), PEG-20 Methyl Glucose Sesquistearate (Glucamate® SSE, HLB = 15) oder Mischungen mit Fettalkoholen wie Montanov® 82 (Cetearyl Alcohol, Coco Glucoside, HLB = 11), Montano® 14 (Myristyl Alkohol, Myristyl Glucoside, HLB = 10), Montanov® 202 (Arachidyl Alcohol, Behenyl Alcohol, Arachidyl Glucoside, HLB = 8) oder Sucroseester wie Sucrose Stearate (Crodesta® F 160, HLB = 14,5), Sucrose Cocoate (Crodesta® SL 40, HLB = 15) bzw. Mischungen hiervon, z.B. Crodesta® F 110 (Sucrose Stearate, Sucrose Distearate, HLB = 12).

Ferner können Sterol-Derivate als Emulgatoren eingesetzt werden, z.B. LanolinDerivate wie Laneth-20 (Polychol® 20, HLB = 14) oder Generol® RE 10 (PEG-10 Rapeseed Sterol, HLB = 12) oder Rizinusöl-Derivate wie PEG-40 Hydrogenated Castor Oil (Eumulgin® HRE 40, HLB = 15) oder PEG-36 Castor Oil (Arlatone® 650, HLB = 12,5), bzw. deren Gemische wie Arlatone® 980 (PEG-15 Hydroxystearate, PEG-25 Hydrogenated Castor Oil, HLB = 12,8).

Es können bevorzugt auch Siloxan-Derivate, insbesondere Polysiloxan-Polyether-Copolymere wie Abil® Care 85 (Dimethicone Copolyol, Caprylic/Capric Triglyceride, HLB = 10) eingesetzt werden.

Als ionische Emulgatoren können hier einwertige Salze z.B. von Fettsäuren oder Fettalkoholsulfaten eingesetzt werden z.B. Natriumstearat oder Triethanolaminostearat, Lanette® E (Sodium Cetearyl Sulfate) oder aber Phosphate wie Amphisol® K (Potassium Cetyl Phosphate) oder Glutamate, z.B. Hostapon® CCG (Sodium Cocoyl Glutamate) oder Lactylate, z.B. Crolactil® SSL (Sodium Stearoyl Lactylate).

Alle oben genannten Emulgatoren können auch in Gemischen mit Co-Emulgatoren, welche auch allgemein als **Konsistenzgeber** verwendet werden können, eingesetzt werden. Diese können aus der Gruppe der Fettalkohole, wie Stearyl Alcohol (Lanette® 18), Cetyl Alcohol (Lanette® 16), Myristyl Alcohol (Lanette® 14) oder Cetearyl Alcohol (Lanette® O) ausgewählt werden. Ferner geeignet sind Fettsäuren, z.B. Stearinsäure oder Glycerylester wie Glycerylstearat, insbesondere Glycerinmonostearat oder Glycerindistearat oder Gemische hiervon, z.B. Tegin® M.

Weiterhin können als Konsistenzgeber auch Wachse eingesetzt werden, z.B. Bienenwachs (Lunacera® alba), Kester®Wachs K82H (C₂₀₋₄₀- Alkylstearat) oder Lunacera® M (Micro Wax) oder Kohlenwasserstoffwachse wie Lunacera® P (Mineral Wax) sowie hydriertes Rizinusöl (Cutina® HR) oder synthetische Wachse wie Cetylpalmitat (Cutina® CP) oder Myristylmyristat (Crodamol® MM), oder Stearylstearate (Crodamol® SS).

Besonders bevorzugte O/W Emulgatoren sind:
Tego Care® 450(Polyglyceryl-3 Methyl Glucose Disterate, HLB = 12),
Eumulgin® B1 (Ceteareth-12, HLB = 13) oder Mischungen hiervon.

Ferner bevorzugt sind auch die o.g. Hostaphat®-Produkte oder Abil® Care 85.

### IV.Zusatzstoffe

### 1) Zusatzwirkstoffe

Als Zusatzwirkstoffe können insbesondere Vitamine wie z.B. Tocopherolacetat (Vitamin E) oder Vitamin A, z.B. als Retinolpalmitat gewählt werden. Ferner sind Elektrolyte wie Magnesiumsulfat oder Natriumchlorid (Elektrolyte z.B. in Mengen von 0,2- 2%) geeignet. Ferner können Polysaccharide, wie Glykosaminglycane, insbesondere Mucopolysaccharide eingesetzt werden. Hierzu gehören insbesondere nicht heparinoide Verbindungen wie z.B. Chondroitinsulfat oder Dermatansulfat oder Keratansulfat oder auch heparinoide Verbindungen wie Heparin, insbesondere deren Salze, z.B. Natriumsalze.

Ferner sind insbesondere Allantoin, D- Panthenol, Hyaluronsäure und/oder Zinkderivate wie Zincidone® (Zink PCA), Zinkglukonat oder Zinkoxid geeignet.

Die Mengen an einzelnen Zusatzwirkstoffen variieren und können z.B. von je jeweils 0,01- 20% bzw. 0,1- 6%, insbesondere 1- 5% bzw. 3- 5% betragen.

Darüber hinaus sind Vesikelbildner, insbesondere Lecithine und Analoge hiervon als Zusatzwirkstoffe geeignet. Hierzu gehören z.B. bekannte Stoffe (vgl. DE 42 05 548 C2), insbesondere Phospholipide wie Lecithin (Ei- oder Soja-Lecithin), z.B. Phosal® 50 SA (ca. 50 % Soja-Lecithin), Phosphatidylcholin, -serin oder - diethanolamin sowie deren Mischungen.

Weitere geeignete Lecithin- analoge Komponenten sind Sphingolipide (z.B. Ceramide, Cerebroside, Sphingosin, Sphingomyelin), Phytosterole (im wesentlichen Gemische aus β-Sitosterol, Campesterol und Stigmasterol) sowie deren Derivate, insbesondere Ethoxylate wie Generol® 122 E 5 (PEG-5 Soybean Sterol), Gererol® R E5 (PEG-5 Rapeseed Sterol).

Ferner geeignet als Vesikelbildner sind polyethoxylierte Fettalkohole sowie Fettsäuren mit vorzugsweise 1-4 EO mit einem HLB-Wert von 2 bis 6 wobei der lipophile Rest bevorzugt aus C₁₆ bis C₁₈-Alkylketten besteht, Polyglycerolalkylether, Glucosyldialkylether, Saccharosediester, Kollagenhydrolysatester, quartäre Ammoniumverbindungen und Poloxamere. Besonders bevorzugt sind Ei- und/oder Soja-Lecithin (Phosal® 50SA), Phosphatidylcholin, Ceramide, Phytosterole und ethoxilierte Derivate hiervon mit einem Ethoxilierungsgrad von 5 bis 16 und polyoxyethylierte Fettalkohole mit einem HLB-Wert von 2-6 oder Kombinationen hiervon, insbesondere von Phytosterolen der o.g. Art mit Ethoxylierungsprodukten hiervon.

Besonders bevorzugt hier ist Phosal® 50 SA.

Insbesondere bevorzugte Zusatzwirkstoffe sind:
a) Vitamin E (z.B. 0,1- 5, insbesondere 0,1- 1%);
b) Magnesiumsulfat und /oder Natriumchlorid (z.B.0,1-5, insbesondere 0,1- 1%);
c) Mucopolysaccharide wie z.B. Chondroitinsulfat (z.B.0,1- 2, insbesondere 0,1-1%) sowie Heparin oder Heparinoide wie Heparin-Na (z.B.0,1-2, insbesondere 0,1-1%);
d) Allantoin; Mengen wie vorstehend; z.B. 0,05-2%, bevorzugt 0,1-1%;
e) D- Panthenol; Mengen wie vorstehend; z.B. 0,1-10%, bevorzugt 1-5%;
f) Hyaluronsäure (z.B.0,001- 1, insbesondere 0,01- 0,1%);
g) Lecithine wie Phosal® 50 SA (z.B.0,5- 5%, insbesondere 1- 2%);
h)Zinkderivate wie Zinkgluconat oder Zink PCA (Zincidone®), z.B. 0,1-3, insbesondere 0,5-1 %
i)ölabsorbierende Substanzen, z.B. Stärke-Derivate wie Natrasorb® HFB (Aluminium Starch, Octenylsuccinate, Acrylates Copolymer, Magnesium Carbonate) oder Acrylate, z.B. Micropearl® M 100 (Polymethyl Methacrylate), Micropearl® M 305 Methyl Methacrylate Copolymer) oder Sulfonate wie Biopol® OE (Sodium C8-16 Isoalkylsuccinyl Lactoglobulin Sulfonate).

Weiterhin können adstringierende und sebumregulierende Substanzen eingesetzt werden wie Acnacidol® 101 (Propylene Glykol, Hydroxydecanoic Acid), Asebiol® BT (Hydrolyzed Yeast Protein, Pyridoxine, Niacinamide, Glycerin, Panthenol, Propylene Glycol, Allantoin, Biotin), Lipacide® C8C0 (Caproyl Collagen Aminoacids), Sebosoft® (Glycerin, Aqua, PEG-8, Caprylyl Glycol, Sebacic Acid, Sodium Polyacrylate), Sepi Control® A5 (Capryloylglycine, Methylglycine, Cinnamonum zeylanicum).

Ferner können als Zusatzwirkstoffe weitere Pflanzenextrakte enthalten sein, z.B. Birkenblätterextrakt, Aloe-Vera-Extrakt, Ringelblumen-, Hibiskus-, Klettenwurzel-, Hamamelis-, Wassernabelkraut-, Algen-, Quitten-, Wasserlilien-, Zimtextrakt.

Es können auch kühlende/beruhigende Wirkstoffe wie Frescolat® ML (Menthyl Lactate) oder Eashave® (Sodium Hyaluronate, Wheat Germ Extrakt, Saccharomyces Cerevisiae Extrakt) inkorporiert werden.

Darüber hinaus sind als Zusatzwirkstoffe durchblutungsfördernde Stoffe, z.B. Nikotinsäurederivate wie Methyl- oder Tocopherylnikotinat, Alpha- und Betahydroxysäuren und deren Derivate, z.B. Glykol-, Äpfel-, Zitronen-, Wein-Milchsäure, Salicylsäure, Isopropylbenzylsalicylate, C12 - 13 Alkyl Lactate (Cosmacol® ELI)oder auch antiphlogistische und antibakterielle Substanzen wie Triterpene, z.B. Ursolsäure, Glycyrrhizinsäure oder Glycyrrhetinsäure und deren Derivate, z.B. Stearyl Glycyrrhetinate, Kaliumglycyrrhinat; Pantothensäurederivate, z.B. D-Panthenol, Panthenyltriacetat; Allantoin; Bisabolol; Azulene, z.B. Cham-Azulene oder Guaj-Azulen; Phytosphingosine; Triclosan; Chlorhexidin-Derivate und/oder Antischuppenmittel, z. B. Climbazol oder Piroctone Olamine einsetzbar.

Daneben können in den erfindungsgemäßen Zusammensetzungen auch antioxidativ sowie zellschützend wirkende Stoffe wie Flavonoide, z.B. Rutin, Ferulasäure und deren Ester oder Isoflavone wie Soja-Isoflavone oder Coenzym Q 10 als wirksame Zusatzstoffe eingesetzt werden.

Ganz besonders bevorzugt werden Zusatzwirkstoffkombinationen aus a)- f) bzw. a)- i) oder a)+ c)- i). Diese können dann in geeigneter Weise mit den nachfolgend beschriebenen üblichen Zusatzstoffen kombiniert werden.

### 2) Übliche Zusatzstoffe

Diese sind vorzugsweise ausgewählt aus:
Antioxidantien, Parfümstoffen, Farbstoffen, UV-Filtern, Konservierungsmitteln und/oder Feuchthaltern, Stabilisatoren, Konsistenzgebern.
Die Antioxidantien können bevorzugt ausgewählt werden aus Butylhydroxytoluol, Butylhydroxyanisol, Ascorbylpalmitat, Tocopherol, evtl. in Kombination mit Synergisten wie in Controx® VP (Tocopherol,Lecithin, Ascorbyl Palmitate, Hydrogenated Palm Glycerides Citrate), Gallussäurealkylester wie Octyl-, Dodecyl- und Cetylgallat oder Kombinationen hiervon.
Parfümstoffe sind insbesondere ausgewählt aus etherischen Ölen. Ferner sind auch handelsübliche Parfüm-Kompositionen möglich wie z.B. Parfümöl Deliana .
Etherische Öle, die für die erfindungsgemäßen Mittel geeignet sind, sind insbesondere die etherischen Öle ausgewählt aus Rosmarinöl, Orangenöl, Lavendelöl, Limettenöl, Zimtöl, Geraniumöl, Zedernholzöl, Rosenholzöl, Baldrianöl, Ylang-Ylang Öl, Citronellöl, Teebaumöl, Manukaöl, Eucalyptusöl, Minzöl, Lemongrasöl, Zypressenöl, Niaouliöl, Fichtennadelöl, Kiefernnadelöl, Campher, Menthol.
Diese Zusatzstoffe können auch als Zusatzwirkstoffe angesehen werden. Hierzu zählen dann auch essentielle ungesättigte Fettsäuren und deren Ester, z.B. Linol- oder Linolensäure, Glyceryl Linoleate, Glyceryl Linolenate eingesetzt werden.
Besonders bevorzugte Parfüm- bzw. Zusatz-Wirkstoffe sind ausgewählt aus etherischen Ölen, Pflanzenextrakten und -ölen, sebumregulierenden Stoffen, Feuchthaltemitteln, antiphlogistischen und antibakteriellen Substanzen, Vitaminen, ungesättigten essentiellen Fettsäuren oder Mischungen hiervon.

Bevorzugte Farbstoffe sind z.B.. Patentblau, Amidoblau, Orange RGL, Cochenillerot, Farbstoff FD+C Blue No.1 oder Titandioxid oder Chinolingelb.

Geeignete UV-Filter sind UVB, UVA und Breitbandfilter der folgenden Art:
UV-B Filter: Zimtsäureester, z.B. Octyl Methoxycinnamate (Eusolex® 2292, Neo Heliopan® AV, Parsol® MCX), Isoamyl p-Methoxycinnamate (Neo Heliopan® Galanga) sowie 4-Methylbenzyliden Camphor (Eusolex® 6300), Paraaminobenzoesäure und -ester wie N, N-Dimethyl-4-aminobenzoesäure-2-ethylhexylester (Eusolex® 6007, Octyl Dimethyl PABA), Homomenthylsacilylat (Homosalate, Eusolex® HMS), Octyl Salicylate (Neo Heliopan® OS), Octocrylene (Neo Heliopan® 303), Phenylbenzimidazolesulfonic Acid (Neo Heolipan® Hydro, Eusolex® 232)
UVA + UVB Filter für Breitbandabsorption wie Benzophenone-3 (Neo® Heliopan BB, Eusolex® 4360);
UV-A Filter wie Methyl Anthranilate (Neo Heliopan® MA), Butyl Methoxydibenzoylmethane (Parsol® 1789, Eusolex® 9020); Methylene Bis-Benzotriazolyl Tetramethylbutylphenol (Tinosorb® M), Bis-Ethylhexyloxyphenol Methoxyphenyltriazin (Tinosorb® S), Disodium Phenyl Dibenzimidazole Tetrasulfonate (Neo Heliopan® AP).

Insbesondere bevorzugt sind Octyl- oder Isoamyl p- Methoxycinnamate, Octocrylene, 4-Methylbenzyliden Camphor, Homosalate und/oder Butyl Methoxydibenzoylmethane und/oder Benzophenone-3.

Weiterhin geeignet sind anorganische UV-Filter wie Zinkoxid und Titandioxid insbesondere mikronisiert und/oder beschichtet, z.B. Z-Cote®, Tioveil®.

Als Konservierungsstoffe kommen lodopropynylbutylcarbamat, DMDM Hydantoin, Phenoxyethanol und weitere gebräuchliche Konservierungsstoffe, wie z.B. Sorbin- und Dehydracetsäure und deren Salze, Methyldibromoglutanonitril, etc. oder Kombinationen hiervon, oder andere Säuren wie Benzoe- oder Salicylsäure, oder Benzylalkohol oder Ester wie p-Hydroxy-benzoesäureester, z.B. Methyl-, Ethyl-, Propyl-, Butyl-, Iso-Butylparaben, bevorzugt Methyl-oder Propylparaben oder Mischungen hiervon oder Climbazol oder geeignete Kombinationen der genannten Stoffe wie z.B. Methyl-, Propylparaben und Sorbinsäure in Frage. Insbesondere geeignet sind Mischungen, z.B. aus DMDM Hydantoin und lodopropynylbutylcarbamat wie Glydant® plus.

Weiterhin geeignet sind Feuchthalter z.B. Polyalkohole wie Polyethylenglykol, Propylenglykol, Butylenglykol, Sorbitol, Glycerol oder Polymere, z.B. Polyquaternium-Typen wie Polyquaternium -39 (Merquat® plus 3330), Proteine wie Collagen oder dessen Hydrolysate, Aminosäuren, Harnstoff, D-Panthenol, pflanzliche Proteine wie z.B. aus Weizen, Soja oder Mandel oder deren Hydrolysate, z.B. Tritisol® (Hydrolyzed Wheat Protein), Polysaccharide wie z.B. Fucogel® 1000 (Biosaccharide Gum-1), Glucosaminoglykane, z.B. Hyaluronsäure oder sulfatierte Glucosaminoglykane wie Chondroitinsulfat, Dermatansulfat, Keratansulfat, Heparansulfat, insbesondere deren Na-Salze oder Heparin-Na , Glukane, z.B. β-Glukan, z.B. Hafer β-Glukan (Drago-β-Glucan®), Mannane wie Konjac Mannane, Algenextrakte, z.B. Seamannin® SU oder handelsübliche Feuchhaltemittel wie z.B. Hydractin® (Glycerin, Aqua, Disodium Adenosine Triphosphate, Algin, Carica Papaya) oder Aquaderm® (Sodium PCA, Sodium Lactate, Fructose, Glycine, Niacinamid, Urea, Inositol), Salze, z.B. Natriumlactat, DL-2-Pyrrolidon-5-Carbonsäure, Na-Salz.

Bevorzugt sind Polyethylen-/ Propylenglykol oder Glycerin in Mengen von z.B.0,5- 10%, insbesondere 2- 5%, sowie Polysaccharid- Verbindungen wie Fucogel® 1000.

Als Stabilisatoren eignen sich zum einen Wachsprodukte wie z. B. Lunacera® alba (Cera alba), Lunacera® M (Cera Microcristallina), Cutina® HR (Hydrogenated Castor Oil) oder Silikonwachse, z.B. Abil® Wax 9800 (Stearyl Dimethicone). Ferner können auch Amerchol® CAB bestehend aus Vaseline und Lanolin, als Stabilisatoren eingesetzt werden.

Zum anderen sind als Stabilisatoren Komponenten zur pH- Wert Regulation verwendbar wie NaOH (z.B. 5% ig, z.B. in Mengen von 0,1- 4%, insbesondere 1-3%) oder Säuren wie z.B. Zitronensäure, Milchsäure oder Apfelsäure oder EDTA- Na in geeigneter Menge als Komplexiermittel. Die Menge und Art der pH-Wert -Regulatoren hängt von den übrigen Zusatzstoffen ab und sind dem Fachmann geläufig.

Insbesondere ist als Stabilisator eine Kombination aus einem oder mehreren Acrylamiden, einem oder mehreren Acrylaten und einem oder mehreren Polysacchariden, insbesondere Stärke, bzw. Stärke-Derivaten geeignet, wobei jede Komponente in einer Menge von 0,05-8%, bevorzugt 0,1-5% enthalten sein kann.

Besonders bevorzugte Acrylamide sind Polyacrylamid, z.B. Flocare® T 920 GC oder Polyacrylamidhaltige Gemische wie Sepigel® 305 (Polyacrylamide, C13-14 Isoparaffin, Laureth-7), Sepigel® 501 (Acrylamides Copolymer, Mineral Oil, C13-14 Isoparaffin, Polysorbate 85), Sepigel® 502 (C13-14 Isoparaffin, Isostearyl Isostearate, Sodium Polyacrylate, Polyacrylamide, Polysorbat 20), Creagel® EZ DC (Polyacrylamide, Polydecene, Dimethicone Copolyol), Creagel® EZ 5 (Polyacrylamide, Polydecene, Laureth-5).

Als Acrylate werden Carboxy-Vinyl-Mischpolymerisate mit hohem Molekulargewicht (1 - 3 Mio.) sowie deren Copolymere eingesetzt, insbesondere nach Neutralisierung durch Alkali. Bevorzugt sind die unter dem INCI-Namen Carbomer bekannten Carbopol®-Typen, z.B. Carbopol® 910, 934, 940, 941, 954, 980, 981, 2984, 5984 oder Carbopol® ETD 2001, 2050 oder Synthalen® K, L, M oder die bereits neutralisierten Carbomere wie PNC® 400, 410, 430 (INCI: Sodium Carbomer). Es können auch Acrylat-Copolymere eingesetzt werden, z.B. Arylates/C10-30 Alkyl Acrylate Crosspolymer, bekannt als Carbopol® 1342, 1382, ETD 2020, Pemulen® TR-1, TR-2.

Als Stärke, bzw. Stärke-Derivate sind insbesondere folgende Stoffe geeignet:
Reis-, Weizen-, Mais- und Kartoffelstärke.

Besonders bevorzugt sind hydrophobisch modifizierte Stärken wie Aluminium Starch Octenylsuccinate (Dry Flo® PC, Fluidamid® DF 12) oder dessen Gemische wie Natrasorb® HFB (Aluminium Starch Octenylsuccinate, Acrylates Copolymer, Magnesium Carbonate), ASO/MM3® (Aluminium Starch Octenylsuccinate, Magnesium Myristate), Dry Flo® Elite LL (Aluminium Starch Octenylsuccinate, Lauroyl Lysine), Facemat® (Aluminium Starch Octenylsuccinate, Mica, Zea Mays (Corn) Starch, Silica, Titanium Dioxide, Zink Oxide).

Ganz besonders bevorzugt sind Dry Flo® PC und Natrasorb® HFB.

Die oben genannte Kombination aus Stabilisatoren kann auch insbesondere bei Einsatz von O/W- Emulgatoren verwendet werden.

Besonders bevorzugt ist hier Sepigel®305 oder 501, PNC®410/400 oder Carbopol®ETD2020 und Dry Flo®PC oder Natrasorb®HFB, insbesondere Kombinationen hiervon.

Bevorzugt werden Konservierungsstoffe, Antioxidantien, Farb- und Parfümstoffe zusammen mit Stabilisatoren und Feuchthaltern und Zusatzwirkstoffen ggf. in Verbindung mit pH- Regulatoren als Zusatzstoffe, insbesondere bevorzugt in Verbindung mit den o.g. Zusatzwirkstoffen eingesetzt.

Die Mengen an Zusatz- bzw. Wirkstoffen können in den angegebenen Bereichen variieren.

Bevorzugt sind Zusatzstoffe ausgewählt aus Zusatzwirkstoffen wie Vitaminen, Elektrolyten wie Magnesiumsulfat und Natriumchlorid, Allantoin, D- Panthenol, Hyaluronsäure, Mucopolysacchariden wie z.B. Heparinoiden und Nicht-Heparinoiden sowie deren Mischungen, sowie Parfümstoffen, Konservierungsmittel und Feuchthaltemittel.

Diese können bevorzugt mit Wachsen und/oder Lecithinen wie insbesondere Phosal®50SA kombiniert werden.

Das beschriebene Zwiebelextrakt- enthaltende Mittel kann hergestellt werden, indem man Zwiebelextrakt , bevorzugt mit dort löslichen Zusatzstoffen vereinigt, sodann die Fettphase herstellt, wobei bevorzugt dort lösliche Zusatzstoffe inkorporiert werden können, und dann die Wasserphase bereitet, welche bevorzugt dort lösliche Zusatzstoffe und insbesondere Alkohole der oben genannten Art und Menge aufweist, und sodann die Wasser- und die Fettphase bei Temperaturen von 60 bis 90°C zusammen mit einem oder mehreren Emulgatoren oder Gemischen hiervon oder mit Co- Emulgatoren emulgiert, ggf. homogenisiert und nach Abkühlen (z.B. bei 20-50°C) die Zusatzstoffe, sofern vorhanden, hinzufügt und in geeigneter Weise aufarbeitet, z.B. durch Homogenisieren.

Andererseits kann der Zwiebelextrakt zusammen mit den Zusatzstoffen in die Fett(öl)phase inkorporiert werden, wobei ggf. auch Lösungsmittel, insbesondere Alkohole der oben genannten Art und Menge hinzugefügt werden können.

Als Zwiebelextrakt wird der jeweils gewünschte wässrige, alkoholische, alkoholisch-lösungsmittelhaltige oder wässrig-alkoholische oder Mischungen hiervon, z.B. im Verhältnis 3:1 bis 1:3 z.B. der beiden letztgenannten , in den angegebenen Mengen gewählt.

Ein wie oben beschriebenes hergestelltes Mittel eignet sich insbesondere z.B. als dermatologische Zusammensetzung zur Pflege, Behandlung oder Vorbeugung von geschädigtem Hautgewebe, insbesondere Narbengewebe, oder erschlafftem Gewebe wie z.B. Schwangerschaftsstreifen, oder auch von geschädigtem Hautgewebe, welches entstanden sein kann aufgrund von Schnittwunden, Operationswunden, Verbrennungen oder durch altersbedingte Degeneration.

Das geschädigte Hautgewebe zeigt schon nach kurzer Zeit eine Verbesserung der vernarbten oder geschädigten Anteile und kann durch die neue Ölphasen enthaltende Formulierung über hinaus noch wesentlich weicher, geschmeidiger und elastischer erhalten werden, als ohne diesen pflegenden, hautregenerierenden Zusatz.

Somit kann das Mittel auch bei Narben nach Schönheitsoperationen oder auch zur Behandlung von Aknenarben überraschenderweise mit überaus regenerativem Erfolg eingesetzt werden.

Die Erfindung wird anhand der nachfolgenden Beispiele näher erläutert.

In den Beispielen 1-6 wurde ein wässrig- ethanolischer Zwiebelextrakt, insbesondere mit einem Anteil von 13-20% Ethanol und in den Beispielen 7,8 ein Alkohol-haltiger, nämlich Ethanol und mittelkettige Trigycerid (ca. 60%) enthaltender Zwiebelextrakt eingesetzt. In den Beispielen 9, 10 ist die Wirksamkeit anhand medizinischer Anwendungsbeobachtungen aufgezeigt.

### Beispiel 1

| | |
|---|---|
| Aqua purificata | 34,7879 |
| Magnesiumsulfat | 0,7000 |
| Arlacel 582 Nena | 8,0000 |
| Arlatone TV | 1,5000 |
| Lunacera alba | 2,0000 |
| Lunacera M | 2,0000 |
| Magnesiumsterat | 2,0000 |
| Amerchol CAB | 5,0000 |
| Cetiol OE | 5,0000 |
| Paraffinum Perliquidum | 10,0000 |
| Cosmacol ELI | 3,0000 |
| Nachtkerzenöl | 2,0000 |
| Tocopherolacetat | 0,5500 |
| Oxynex 2004 | 0,0500 |
| Glydant plus | 0,2000 |
| Propylenglykol | 4,0000 |
| D- Panthenol | 5,5000 |
| Allantoin | 1,0000 |
| Hyaluronsäure | 0,0100 |
| Chondroitinsulfat | 0,2000 |
| Zwiebelextrakt | 10,0000 |
| Farbstoff Pigmentpaste | 2,0000 |
| Farbstoff FD+C Blue No. 1 | 0,0003 |
| Parfümöl Deliana | 0,5000 |

### Beispiel 2

| | |
|---|---|
| Aqua purificata | 51,7879 |
| Natriumchlorid | 0,5000 |
| Abil EM 90 | 2,5000 |
| Abil Wax 9800 | 1,0000 |
| Jojobaöl | 1,0000 |
| Arlamol HD | 5,0000 |
| Cetio OE | 4,0000 |
| Lunacera M | 0,6000 |
| Cutina HR Powder | 0,4000 |
| Cosmacol ELI | 3,0000 |
| Nachtkerzenöl | 2,0000 |
| Tocopherolacetat | 0,5500 |
| Oxynex 2004 | 0,0500 |
| Dow Corning 345 Fluid | 4,0000 |
| Glydant plus | 0,2000 |
| Propylenglykol | 4,0000 |
| D- Panthenol | 5,5000 |
| Allantoin | 1,0000 |
| Phosal SA 50 Neu | 2,0000 |
| Hyaluronsäure | 0,0100 |
| Chondroitinsulfat | 0,4000 |
| Zwiebelextrakt | 10,0000 |
| Farbstoff FD+C Blue No. 1 | 0,0002 |
| Parfümöl Deliana | 0,5000 |

### Beispiel 3

| | |
|---|---|
| Aqua purificata | 38,7900 |
| Carbopol ETD 2020 | 0,2500 |
| Tego Care 450 | 3,0000 |
| Eumulgin B1 | 2,0000 |
| Lunacera alba | 2,0000 |
| Kester Wachs K82H | 3,0000 |
| Arlamol HD | 1,5000 |
| Cetiol SB 45 | 2,0000 |
| Finsolv TN | 3,0000 |
| Dow Corning Fluid 345 | 2,0000 |
| Cosmacol ELI | 3,0000 |
| Nachtkerzenöl | 2,0000 |
| Tocopherolacetat | 0,5500 |
| Oxynex 2004 | 0,0500 |
| Propylenglykol | 4,0000 |
| Glydant plus | 0,2000 |
| NaOH 5%ig | 2,8000 |
| Trilon BD | 0,1000 |
| Panthenol | 5,5000 |
| Hyaluronsäure Na- Salz | 0,0100 |
| Phosal SA 50 | 2,0000 |
| Allantoin | 1,0000 |
| Zincidone | 0,5000 |
| Fucogel 1000 | 5,0000 |
| Natrasorb HFB | 3,0000 |
| Zwiebelfluidextrakt | 10,0000 |
| Farbstoff Pigment Paste weiß | 2,0000 |
| Sepigel 305 | 0,5000 |
| Parfümöl Deliana | 0,5000 |

### Beispiel 4

| | |
|---|---|
| Aqua purificata | 33,5900 |
| Tego Care 450 | 3,0000 |
| Eumulgin B1 | 2,0000 |
| Lunacera alba | 1,0000 |
| Kester Wachs K82H | 3,0000 |
| Arlamol HD | 2,5000 |
| Cetiol SB 45 | 2,0000 |
| Finsolv TN | 3,0000 |
| Dow Corning Fluid 345 | 2,0000 |
| Cosmacol ELI | 3,0000 |
| Nachtkerzenöl | 2,0000 |
| Tocopherolacetat | 0,5500 |
| Oxynex 2004 | 0,0500 |
| PNC410 | 0,2000 |
| Propylenglykol | 4,0000 |
| Glydant plus | 0,9000 |
| Panthenol | 5,5000 |
| Hyaluronsäure Na- Salz | 0,0100 |
| Phosal SA 50 | 2,0000 |
| Allantoin | 1,0000 |
| Zincidone | 0,5000 |
| Fucogel 1000 | 5,0000 |
| Sepigel305 | 0,5000 |
| Zwiebelfluidextrakt | 10,0000 |
| Farbstoff Pigment Paste weiß | 2,0000 |
| Dry Flow PC | 5,0000 |
| Parfümöl Deliana | 0,5000 |

### Beispiel 5

| | |
|---|---|
| Aqua purificata | 41,0900 |
| Carbopol ETD2020 | 0,2000 |
| Dermofeel Q182S | 3,0000 |
| Eumulgin B1 | 2,0000 |
| Lunacera alba | 2,0000 |
| Kester Wachs K82H | 3,0000 |
| Arlamol HD | 5,0000 |
| Cetiol SB 45 | 2,0000 |
| Finsolv TN | 3,0000 |
| Dow Corning Fluid 345 | 2,0000 |
| Cosmacol ELI | 3,0000 |
| Tocopherolacetat | 0,5500 |
| Oxynex 2004 | 0,0500 |
| Propylenglykol | 4,0000 |
| Glydant plus | 0,2000 |
| NaOH 5%ig | 1,6000 |
| Panthenol | 5,5000 |
| Hyaluronsäure Na- Salz | 0,0100 |
| Phosal SA 50 | 2,0000 |
| Allantoin | 1,0000 |
| Zincidone | 0,5000 |
| Fucogel 1000 | 5,0000 |
| Sepigel305 | 1,0000 |
| Zwiebelfluidextrakt | 5,0000 |
| Farbstoff Pigment Paste weiß | 1,0000 |
| Parfümöl Deliana | 0,5000 |
| Natrasorb HFB | 3,0000 |

### Beispiel 6

| | |
|---|---|
| Aqua purificata | 46,1900 |
| Carbopol ETD2020 | 0,2000 |
| Dermofeel Q182S | 3,0000 |
| Eumulgin B1 | 2,0000 |
| Lunacera alba | 2,0000 |
| Kester Wachs K82H | 1,0000 |
| Arlamol HD | 5,0000 |
| Cetiol SB 45 | 2,0000 |
| Finsolv TN | 3,0000 |
| Dow Corning Fluid 345 | 2,0000 |
| Cosmacol ELI | 3,0000 |
| Tocopherolacetat | 0,5500 |
| Oxynex 2004 | 0,0500 |
| PNC 410 | 0,6000 |
| Propylenglykol | 4,0000 |
| Glydant plus | 0,2000 |
| NaOH 5%ig | 1,6000 |
| Panthenol | 5,5000 |
| Hyaluronsäure Na- Salz | 0,0100 |
| Phosal SA 50 | 2,0000 |
| Allantoin | 1,0000 |
| Zincidone | 0,5000 |
| Fucogel 1000 | 5,0000 |
| Sepigel305 | 0,3000 |
| Zwiebelfluidextrakt | 5,0000 |
| Farbstoff Pigment Paste weiß | 1,0000 |
| Parfümöl Deliana | 0,5000 |
| Natrasorb HFB | 3,0000 |

### Beispiel 7

| | |
|---|---|
| Miglyol 812 | 73,9000 |
| Zwiebelextrakt | 2,0000 |
| Tocopherolacetat | 1,1000 |
| Nachtkerzenöl | 2,0000 |
| Jojobaöl | 5,0000 |
| Cosmacol ELI | 5,0000 |
| Cetiol PGL | 10,0000 |
| Parfümöl Deliana | 1,0000 |

### Beispiel 8

| | |
|---|---|
| Miglyol 812 | 86,9000 |
| Zwiebelextrakt | 2,0000 |
| Tocopherolacetat | 1,1000 |
| Nachtkerzenöl | 2,0000 |
| Jojobaöl | 3,0000 |
| Cosmacol ELI | 5,0000 |
| Parfümöl Deliana | 0,7000 |

### Beispiel 9 Medizinische Anwendungsbeobachtungen mit Patienten mit unterschiedlichsten Narben

Bei 338 Patienten (62 % weiblich) im durchschnittlichen Alter von 41 Jahren waren Schnittwunden (45), Schürfwunden (14), Operationswunden (240), Verbrennungsnarben (18), Aknenarben (6) oder andere Narben (15) im Kopf- /Halsbereich (65), Armen (135), Beinen (84), am Oberkörper/Brust (27), Oberkörper/Rücken (14) oder anderen Stellen (54) über einen Zeitraum von < 1 Monat (106) bis 3 Monate (117) bzw. > 3 Monate vorhanden. Diese Patienten wurden über einen Zeitraum bis 6 Monate mit einem Produkt gemäß vorliegendem Beispiel 1 durch Auftragen auf die betroffene Hautstelle im Durchschnitt 2x täglich behandelt. Vor der Behandlung war in etwa 15 % der Fälle eine Anwendung bekannter Produkte wie Zwiebelextrakt-Gel (Contractubex) oder Ketofibrase ohne weiteren Erfolg vorgenommen worden. Während der erfindungsgemäßen Behandlung erfolgte eine Beurteilung hinsichtlich der Hautglättung (Aussehen der Narbe wie übrige Haut bzw. leicht oder stark verhärtet, Reduktion Narbentiefe, -länge), der Hautverträglichkeit (Verteilbarkeit, Einziehvermögen, Geruch, Hautgefühl). Die Ergebnisse sind in nachfolgender Tabelle 1 dargestellt und zeigen, dass das erfindungsgemäße Produkt bei über 90% der Fälle zu einem positiven Ergebnis geführt hatte.

**Tabelle 1**

| Narbenglättung: | Beurteilung Arzt | Beurteilung Patient |
|---|---|---|
| a) Sehr gut | 45,0 % | 46,7 % |
| b) gut | 47,6 % | 44,7 % |
| c) mäßig | 5,6 % | 8,0 % |
| *Gesamt-Urteil a) + b)* | *91,4 %* | *92,6 %* |

| Hautverträglichkeit: | | |
|---|---|---|
| a) sehr gut | 75,9 % | 70,3 % |
| b) gut | 23,2 % | 28,5 % |
| c) mäßig | 0,9 % | 1,2 % |
| *Gesamt-Urteil a) + b)* | *99,1 %* | *98,8 %* |

### Beispiel 10: Medizinische Anwendungsbeobachtung bei der Vorbeugung und Behandlung von Schwangerschaftsstreifen

741 Probanden wurden ab der 16. Schwangerschaftswoche im Hinblick auf die Ausbildung, Verhinderung von Schwangerschaftssteifen über einen Zeitraum von 5-7 Monaten bis 8 Wochen nach der Geburt beobachtet und mit einem Produkt gemäß Beispiel 2 oben auf Bauch, Brust und Oberschenkel 1-2x täglich behandelt. Zu beginn der Studie lagen bei 62,5 % keine Streifen, bei 37, 3 % Streifen (davon 19,2 % leicht, 14,7 % viele leichte, 3,4 % viele starke) vor. Von den Probandinnen waren 65,8 % Erstgebärende (davon 7 % mit Steifen) und 34,1 % Mehrfachgebärende (davon 29,1 % mit Streifen). Nach dem Beobachtungszeitraum ergab sich, dass trotz einer durchschnittlichen Gewichtszunahme von ca. 12 kg bei 35.9 % keine Schwangerschaftsstreifen sich entwickelten und nur bei 37,1 % wenige leichte Streifen auftraten, während bei 21,3 % viele leichte und nur bei 5,7 % starke Streifen festgestellt wurden. Das erfindungsgemäße Produkt ist daher außerordentlich gut geeignet zur wirksamen Verhinderung bzw. Reduktion von Schwangerschaftsstreifen, was sich aus den in Tabelle 2 genannten Beurteilungswerten (Kriterien analog Beispiel 9) eindeutig ergibt.

**Tabelle 2**

| Entwicklung Streifen: | Beurteilung Arzt | Beurteilung Patient |
|---|---|---|
| a) Sehr gut | 53,0 % | 51,1 % |
| b) gut | 39,0 % | 40,1 % |
| c) mäßig | 7,0 % | 6,0 % |
| d) schlecht | 1,0 % | 2,3 % |
| *Gesamt-Urteil a) + b)* | *92,0 %* | *91,1 %* |

| Hautverträglichkeit: | Beurteilung Arzt | Beurteilung Patient |
|---|---|---|
| a) sehr gut | 69,6 % | 75,7 % |
| b) gut | 28,1 % | 22,9 % |
| c) mäßig | 0,9% | 0,9 % |
| *Gesamt-Urteil a) + b)* | *97,7 %* | *98,6 %* |

## Patentansprüche

1. Eine Fett(öl)-phase und einen Zwiebelextrakt aufweisende Creme, Balsam, Fluid oder Lotion, **dadurch gekennzeichnet, dass** diese 2-15 Gew. % Zwiebelextrakt, 0,1-25% Zusatzstoffe, ausgewählt aus Konsistenzgebern, Farbstoffen, Parfümstoffen, Feuchthaltern, Antioxidantien, Konservierungsmitteln, Stabilisatoren, Zusatzwirkstoffen, 1-8 Gew. % Emulgatoren sowie Wasser oder Wasser / Alkohol aufweisen.

2. Creme, Balsam, Fluid oder Lotion gemäß Anspruch 1, **dadurch gekennzeichnet, dass** diese ein oder mehrere W/O- oder O/W Emulgatoren oder Mischungen mit Co- Emulgatoren aufweisen.

3. Creme, Balsam, Fluid oder Lotion gemäß Anspruch 1 oder 2, **dadurch gekennzeichnet, dass** der Zwiebelextrakt ein Wasser, Alkohol oder Wasser-Alkohol enthaltender Zwiebelextrakt ist.

4. Creme, Balsam, Fluid oder Lotion gemäß einem der Ansprüche 1-3, **dadurch gekennzeichnet, dass** der W/O- Emulgator einen HLB- Wert von 1-8 und der O/W Emulgator einen HLB- Wert von 9-18 aufweist oder ein ionischer O/W- Emulgator ist.

5. Creme, Balsam, Fluid oder Lotion gemäß einem der Ansprüche 1-4, **dadurch gekennzeichnet, dass** diese als Emulgator Sorbitan-Derivate, polyethoxylierte, Fettsäuren/Alkohole/Ester/ Triglyceride, (Poly)glycerylderivate, Polyolester, Glucose-Derivate, Pentaerythrit- Derivate, Alkyl-phenole, (Block)Polymere, Siloxane, Fettsäuresalze oder Mischungen hiervon enthalten.

6. Creme, Balsam, Fluid, Lotion, gemäß einem der Ansprüche 1-5, **dadurch gekennzeichnet, dass** die Öl(Fett)phase ausgewählt ist aus Kohlenwasserstoffen, Fettalkoholen, Fettalkoholethern oder -estern, (Polyol)fettsäureestern, Triglyceriden, natürlichen Ölen , natürlichen Fetten, Wachsen, Silikonölen, Silikonwachsen oder Mischungen hiervon.

7. Creme, Balsam, Fluid, Lotion gemäß einem der Ansprüche 1-6, **dadurch gekennzeichnet, dass** diese als ölphase flüssige Paraffine, Milchsäureester, Fettalkoholether, Nachtkerzenöl, Silikonöl oder Mischungen hiervon aufweisen.

8. Creme, Balsam, Fluid, Lotion gemäß einem der Ansprüche 1- 7, **dadurch gekennzeichnet, dass** diese als Zusatzstoffe, solche ausgewählt aus Vitaminen, Elektrolyten, Allantoin, D- Panthenol, Hyaluronsäure, Mucopolysacchariden, aufweisen.

9. Creme, Balsam, Fluid, Lotion gemäß einem der Ansprüche 1- 8, **dadurch gekennzeichnet, dass** diese zusätzlich Wachsprodukte aufweisen.

10. Nicht therapeutische Verwendung einer Creme, Balsam, Fluid, Lotion gemäß einem der Ansprüche 1-9 zur Pflege von geschädigtem Hautgewebe.

11. Verwendung gemäß Anspruch 10, **dadurch gekennzeichnet, dass** das geschädigte Gewebe Narbengewebe, Schwangerschaftsstreifen, degenerative Hautveränderungen, geschädigtes Gewebe nach Operationen, Biopsien, Schnittwunden, Verbrennungen ist.

12. Verwendung einer Creme, Balsam, Fluid, Lotion gemäß einem der Ansprüche 1- 9 zur Herstellung von Zusammensetzungen zur Behandlung oder Vorbeugung von geschädigtem Hautgewebe.

13. Verwendung gemäß Anspruch 12, **dadurch gekennzeichnet, dass** das geschädigte Hautgewebe Narbengewebe ist.

14. Verwendung gemäß Anspruch 13, **dadurch gekennzeichnet, dass** das Narbengewebe solches infolge von Akne oder Schönheitsoperationen entstandenes Gewebe ist.

15. Verwendung gemäß Anspruch 12, **dadurch gekennzeichnet, dass** das geschädigte Hautgewebe Schwangerschaftsstreifen sind.

16. Verwendung gemäß Anspruch 12, **dadurch gekennzeichnet, dass** das geschädigte Hautgewebe solches entstanden aufgrund von Schnittwunden, Operationswunden, Verbrennungen oder durch altersbedingte Degeneration entstandenes geschädigtes Hautgewebe ist.

## Claims

1. A cream, balsam, fluid or lotion comprising a fat (oil) phase and an onion extract, **characterized in that** it contains 2-15% by weight onion extract, 0,1 - 25% by weight additives chosen from consistency-imparting agents, dyes, perfume substances, humectants, antioxidants, preservatives, stabilizers, additional active ingredients, 1-8% by weight emulsifiers and water or water/ alcohol.

2. Cream, balsam, fluid or lotion as claimed in claim 1, **characterized in that** it comprises one or more W/O or O/W emulsifiers or mixtures with coemulsifiers.

3. Cream, balsam, fluid or lotion as claimed in claim 1 or 2, **characterized in that** the onion extract is an onion extract containing water, alcohol or water-alcohol.

4. Cream, balsam, fluid or lotion as claimed in any of claims 1-3, **characterized in that** the W/O emulsifier has a HLB value of 1-8 and the O/W emulsifier has a HLB value of 9-18, or is an ionic O/W emulsifier.

5. Cream, balsam, fluid or lotion as claimed in any of claims 1-4, **characterized in that** it comprises sorbitan derivatives, polyethoxylated fatty acids/alcohols/esters/triglycerides,(poly)-glyceryl- derivatives, polyolesters, glucose derivatives, pentaerythritol derivatives, alkylphenols, (block)polymers, siloxanes, fatty acid salts or mixtures thereof as the emulsifier.

6. Cream, balsam, fluid or lotion as claimed in any of claims 1-5, **characterized in that** the oil(fat) phase is chosen from hydrocarbons, fatty alcohols, fatty alcohol ethers and -esters, (polyol)fatty acid esters, triglycerides, natural oils, natural fats, waxes, silicone oils, silicone waxes or mixtures thereof.

7. Cream, balsam, fluid or lotion as claimed in any of claims 1-6, **characterized in that** it has liquid paraffins, lactic esters, fatty alcohol ethers, evening primrose oil, silicone oil or mixtures thereof as the oil phase.

8. Cream, balsam, fluid or lotion as claimed in any of claims 1-7, **characterized in that** it has, as additives, those chosen from vitamins, electrolytes, allantoin, D-panthenol, hyaluronic acid, mucopolysaccharides.

9. Cream, balsam, fluid or lotion as claimed in any of claims 1-8, **characterized in that** it additionally has wax products.

10. Non- therapeutic use of a cream, balsam, fluid or lotion as claimed in any of claims 1-9 for caring for damaged skin tissue.

11. The use as claimed in claim 10, **characterized in that** the damaged skin tissue is scarred tissue, stretch marks, damaged skin tissue which has arisen as a result of age-related degeneration, damaged tissue which has arisen due to operation wounds, biopsies, cuts, or burns.

12. Use of a cream, balsam, fluid or lotion as claimed in any of claims 1-9 for preparing a medicament for treating or preventing damaged skin tissue.

13. The use as claimed in claim 12, **characterized in that** the damaged skin tissue is scarred tissue.

14. The use as claimed in claim 13, **characterized in that** the scarred tissue is tissue which has arisen as a result of acne or cosmetic operations.

15. The use as claimed in claim 12, **characterized in that** the damaged skin tissue is stretch marks.

16. The use as claimed in claim 12, **characterized in that** the damaged skin tissue is that which has arisen due to cuts, operation wounds, burns or is damaged skin tissue which has arisen as a result of age- related degeneration.

## Revendications

1. Crème, baume, liquide ou lotion présentant une phase grasse (huileuse) et un extrait d'oignon **caractérisés en ce qu'**ils présentent de 2 à 15% en poids d'extrait d'oignon, 0,1 - 25% en poids des additifs choisis parmi des agents de consistance, des colorants, des substances parfumées, des agents humectants, des antioxydants, des conservateurs, des stabilisateurs, des substances actives ajoutées, 1-8 % en poids d' émulsifiants ainsi que de 1' eau ou de l'eau / alcool.

2. Crème, baume, liquide ou lotion selon la revendication 1, **caractérisés en ce qu'**ils présentent un ou plusieurs émulsifiants E/H ou H/E ou des mélanges avec des co-émulsifiants.

3. Crème, baume, liquide or lotion selon la revendication 1 ou 2, **caractérisés en ce que** l'extrait d'oignon est un extrait d'oignon contenant de l'eau, de l'alcool ou de l'eau/alcool.

4. Crème, baume, liquide ou lotion selon l'une des revendications 1 a 3, **caractérisés en ce que** l'émulsifiant E/H présente une valeur d'équilibre hydrophile/lipophile HLB de 1 à 8 et **en ce que** l'émulsifiant H/E présente une valeur HLB de 9 à 18 ou est un émulsifiant H/E ionique.

5. Crème, baume, liquide ou lotion selon l'une des revendications 1 à 4, **caractérisés en ce qu'**ils contiennent comme émulsifiant des dérivés de sorbitane, des acides/alcools/esters gras/ triglycérides polyéthoxylés, des dérivés de (poly)glycéryle, des esters de polyols, des dérivés de glucose, des dérivés de pentaérythritol, des alkylphénols, des polymères (séquencés), des siloxanes, des sels d'acides gras ou des mélanges de ces substances.

6. Crème, baume, liquide ou lotion selon l'une des revendications 1 à 5, **caractérisés en ce que** la phase huileuse (grasse) est choisie parmi des hydrocarbures, des alcools gras, des éthers ou esters d'alcools gras, des esters d'acides gras avec des polyols, des triglycérides, des huiles naturelles, des matières grasses naturelles, des cires, des huiles de silicone, des cires de silicone ou des mélanges de ces substances.

7. Crème, baume, liquide ou lotion selon l'une des revendications 1 à 6, **caractérisés en ce qu'**ils présentent comme phase huileuse des paraffines liquides, des esters de l'acide lactique, des éthers d'alcools gras, de l'huile de carthame, de l'huile de silicone ou des mélanges de ces substances.

8. Crème, baume, liquide ou lotion selon l'une des revendications 1 à 7, **caractérisés en ce qu'** ils présentent comme additifs ceux choisis parmi des vitamines, des électrolytes, l'allantoïne, le D-panthénol, l'acide hyaluronique, des mucopolysaccharides,

9. Crème, baume, liquide ou lotion selon l'une des revendications 1 à 7, **caractérisés en ce qu'** ils présentent en plus des produits à base de cires.

10. Utilisation non thérapeutique d'une crème, d' un baume, d' un liquide ou d' une lotion selon l'une des revendications 1 à 9 destinés à soigner des lésions du tissu cutané.

11. Utilisation selon la revendication 10, **caractérisée en ce que** la lésion du tissu cutané est un tissu cicatriciel, des vergetures de grossesse, un tissu cutané résultant , d'une dégénérescence liée à l'âge, un tissu cutané résultant d'opérations, des biopsies, de plaies de coupure ou de brûlures.

12. Utilisation d'une crème, d' un baume, d' un liquide ou d' une lotion selon l'une des revendications 1 à 9 pour préparer des composition destinées au traitement ou à la prévention des lésions du tissu cutané.

13. Utilisation selon la revendication 12 **caractérisée en ce que** 1 la lésion du tissu cutané est un tissu cicatriciel.

14. Utilisation selon la revendication 13, **caractérisée que** le tissu cicatriciel est un tissu cutane résultant de l'acné ou d'interventions de chirurgie esthétique.

15. Utilisation selon la revendication 12 **caractérisée en ce que** les lésions du tissu cutané sont des vergetures de grossesse.

16. Utilisation selon la revendication 12, **caractérisée en ce que** la lésions du tissu cutané est un tissu cutané résultant de plaies de coupure, de plaies d'opérations, de brûlures, ou d'une dégénérescence liée à l'âge.
